# FASCICULE DE BREVET EUROPEEN

(11) **EP 1 094 054 B1**
(45) Date de publication et mention de la délivrance du brevet: **02.04.2003**
(21) Numéro de dépôt: 00402858.5
(22) Date de dépôt: 16.10.2000
(51) Int. Cl.: C07C 59/08, A61K 7/00, A61K 31/191, C12P 1/04, C12P 7/56, C07C 51/47, C07C 51/42

(54) **Procédé de séparation et de purification d'acide lactique à partir d'un milieu de fermentation**
Verfahren zur Trennung und Reinigung von Milchsäure aus einem Fermentationsmedium
Process for the separation and purification of lactic acid from a fermentation medium

(30) Priorité: 18.10.1999 FR 9912972
(43) Date de publication de la demande: 25.04.2001
(73) Titulaire: Roquette Frères, 62136 Lestrem (FR)
(72) Inventeur: Caboche, Jean-Jacques, 62131 Drouvin le Marais (FR); Fouache, Catherine, 62113 Sailly Labourse (FR); Choque, Jean-Christophe, 59000 Lille (FR); Duflot, Pierrick, 62136 La Couture (FR); Dubois, Eric, 62136 Lestrem (FR)
(74) Mandataire: Boulinguiez, Didier

(56) Documents cités:
- EP-A- 0 393 818
- EP-A- 0 483 831
- WO-A-96/41021
- US-A- 5 641 406
- US-A- 5 814 498

## Description

La présente invention est relative à un procédé particulier de séparation et de purification d'acide lactique à partir d'un milieu de fermentation.

En particulier, la présente invention concerne un procédé de préparation d'acide lactique dont la qualité permet son utilisation non seulement dans des secteurs tels que les domaines d'applications alimentaires, des industries chimiques, agrochimiques, des matières plastiques, des cosmétiques mais surtout dans les domaines d'applications pharmaceutiques.

La préparation d'acide lactique s'effectue habituellement par fermentation de microorganismes des genres *Lactobacillus* (*L. acidophilus, L. delbrüeckii, L. pentosus...*), *Rhizopus, Bacillus* (*B. coagulans...*), *Streptococcus ...*

Cependant, il est connu que la croissance de la plupart de ces micro-organismes producteurs d'acide lactique, voire leur viabilité, est inhibée par la baisse du pH du milieu de fermentation, cette acidification forte du milieu étant provoquée par la production d'acides organiques, dont l'acide lactique lui-même.

Il est donc nécessaire de réguler le pH, et il est généralement admis qu'il faut le maintenir à une valeur comprise entre 4 et 7, de préférence supérieure à 4,5, par exemple comprise entre 5,5 et 6,5, par l'addition de bases , tels les hydroxydes de métaux alcalins ou alcalinoterreux, les carbonates ou les bicarbonates.

L'acide lactique se trouve donc dans ces milieux de fermentation sous forme de sels (lactates de sodium, de potassium, de calcium ou d'ammonium, seuls ou en mélange, en fonction de la base choisie pour réguler le pH du milieu de fermentation).

De ce fait, toutes les méthodes de récupération de l'acide lactique à partir des milieux de fermentation doivent résoudre les mêmes difficultés, i.e. effectuer la séparation entre sel(s) de l'acide lactique, les microorganismes qui les ont produits et les impuretés du milieu de fermentation (sucres et protéines non consommés, et sels inorganiques de natures diverses), et de plus, effectuer la conversion des sels de l'acide lactique en acide lactique sous forme libre, ce qui nécessite également ensuite l'élimination de la base générée correspondante.

Diverses méthodes ont été proposées, pour la récupération de l'acide lactique à partir d'un milieu de fermentation.

Il est connu en toute généralité de purifier l'acide lactique à partir de lactates par ajout d'acide sulfurique.

Par exemple, si la régulation du pH du milieu de fermentation est réalisée par des carbonates ou des bicarbonates, l'acide lactique sous forme libre peut être récupéré par acidification du milieu à l'acide sulfurique.

La réaction donne lieu à la formation de sulfates de calcium (gypse) qui précipitent et à la libération de l'acide lactique sous forme libre qui peut être ensuite extrait par solvant organique, ou adsorbé sur un support adapté et ensuite désorbé.

Cependant, l'inconvénient de cette méthode par ailleurs efficace en termes de rendement, est la consommation élevée d'acide sulfurique, et surtout la production d'importantes quantités de gypse qui pose de sérieux problèmes en termes de traitement des déchets et de biodégrabilité pour l'environnement.

Le brevet JP 63/188.632 décrit la régulation du pH du milieu de fermentation avec de l'hydroxyde de zinc, afin d'obtenir un lactate de zinc de faible solubilité qui autorise sa purification par cristallisation.

L'inconvénient majeur réside ici dans la nécessité d'avoir recours à du sulfure d'hydrogène qui n'est pas aisément manipulable, et ce procédé conduit surtout à de faibles rendements de cristallisation par perte excessive de produit dans la liqueur mère.

Un autre procédé consiste à réaliser une adsorption / désorption avec une tri-alkyl amine. Le brevet DE 2.329.480 décrit par exemple un procédé d'extraction liquide - liquide (ou LLE), qui consiste à extraire l'acide lactique au moyen d'un solvant organique non miscible à l'eau, en présence d'au moins une base de Lewis telle qu'une amine tertiaire. L'acide lactique doit être récupéré dans une seconde étape par une extraction liquide - liquide en retour, qui permet de retransférer l'acide lactique dans l'eau.

L'inconvénient réside cependant dans la formation de lactates de tri-alkyl ammonium ayant des valeurs de pKa d'environ 9 à 11. L'énergie nécessaire pour libérer la forme acide est donc rédhibitoire.

Par extension, tous les procédés nécessitant la décomposition de sels de tri-alkyl ammonium en acides correspondants sont fortement consommateurs d'énergie.

Par ailleurs, Il est décrit l'utilisation de NH₄OH ou de NaOH pour réguler le pH du milieu de fermentation, ce qui permet d'éviter d'avoir recours à l'acide sulfurique et de récupérer plus proprement les lactates sous forme de lactates d'ammonium ou de sodium. Il est possible de procéder ensuite à une étape d'ultrafiltration pour séparer lesdits lactates des autres composants du milieu de fermentation, et enfin d'obtenir l'acide lactique en mettant en oeuvre une étape de séparation sur résines échangeuses d'ions.

Ce procédé met en oeuvre des méthodes membranaires et d'échanges d'ions qui ne permettent pas d'obtenir de l'acide lactique de haute pureté.

Pour remédier à cette difficulté, deux approches ont été développées : une première approche fait appel à la séparation chromatographique, notamment sur résine échangeuse de cations forts; la seconde fait appel à la séparation par électrodialyse de concentration ou dessalement et/ou par électrodialyse fractionnante bipolaire.

Selon la première approche, Il a été proposé de mettre en contact directement le milieu renfermant par exemple du lactate de sodium avec une résine échangeuse de cations forts de type hydrogène, sous forme liquide, pour éliminer les ions sodium.

Mais ce procédé consomme de grandes quantités de résines et d'acides chlorhydrique ou sulfurique pour régénérer ladite résine, et est difficilement utilisable industriellement.

Le brevet EP 483.831 présente une alternative à ce procédé en réalisant la séparation entre un acide organique (ou des acides organiques) et des sels inorganiques, par la mise en oeuvre de colonnes de chromatographie.

Le procédé décrit consiste à cultiver un microorganisme producteur d'acide lactique dans un milieu de fermentation renfermant du glucose comme source carbonée, et de chromatographier, dans des conditions particulières, ledit milieu de fermentation renfermant outre un acide organique (ou des acides organiques), les sucres, les sels inorganiques et autres impuretés résiduelles.

Le procédé consiste plus précisément à passer ledit milieu de fermentation de départ contenant l'acide organique (ou les acides organiques) sur une colonne renfermant la résine échangeuse de cations, ce qui conduit à la rétention dudit acide organique (ou desdits acides) sur la colonne. Ensuite, on fait passer un éluent de type acide minéral pour désorber tout acide organique. Dans le cas où l'on veut séparer plusieurs acides organiques (tels l'acide tartrique et l'acide gluconique) une étape supplémentaire consiste à séparer les acides à partir de l'éluat ainsi obtenu.

Il est décrit qu'il faut impérativement se placer à une valeur de pH inférieure au pKa de l'acide considéré.

Plus particulièrement, il est exemplifié un procédé de préparation de l'acide lactique à partir d'un milieu de fermentation de *L. delbrüeckii*, où le pH du milieu est régulé à 5,8 avec NaOH.

Ledit milieu renfermant 71 g/l de lactate de sodium est débarrassé de ses microorganismes, puis acidifié à un pH de 1,6 avec de l'acide sulfurique. Le passage de ladite solution acidifiée est ensuite effectué sur une colonne de chromatographie dont la résine cationique a été préalablement équilibrée à un pH de 3,09 par passage d'acide sulfurique dilué. L'élution est ensuite réalisée avec encore de l'acide sulfurique, toujours à un pH inférieur au pKa de l'acide lactique, soit inférieur à 3,87, permettant de récupérer une fraction contenant le sulfate de sodium, et une autre fraction contenant l'acide lactique.

Mais ce procédé nécessite d'utiliser encore de grandes quantités d'acide sulfurique à la fois pour abaisser le pH du milieu de fermentation, maintenir le pH dans la colonne de chromatographie mais également ajuster le pH de l'éluant à une valeur inférieure au pKa de l'acide lactique.

Ces quantités d'acide sulfurique seront d'autant plus élevées qu'il sera souhaité d'abaisser la valeur du pH au plus bas, de manière non seulement à limiter la proportion des sels de l'acide lactique en équilibre avec l'acide lactique libre formé, mais également à limiter la perte d'acide lactique libre avec les impuretés (sucres, protéines et sels inorganiques résiduels du milieu de fermentation) qui sont normalement éluées dans les premières fractions de la séparation chromatographique, comme il est par ailleurs déploré dans le brevet EP 483.831.

Selon la seconde approche, il est plutôt procédé à la mise en oeuvre d'une séparation par électrodialyse directement sur le milieu de fermentation contenant les lactates.

C'est ainsi que le brevet EP 393.818 décrit un procédé consistant à soumettre le milieu de fermentation débarrassé des microorganismes à une première électrodialyse conventionnelle pour récupérer et concentrer les sels de l'acide lactique en solution aqueuse débarrassés des impuretés azotées, puis soumettre ladite solution aqueuse à une seconde électrodialyse, cette fois de fractionnement, pour récupérer l'acide lactique libre et la base correspondante. Il faut ensuite traiter la solution renfermant l'acide lactique libre sur un échangeur d'ions forts sous forme acide pour éliminer les ions sodium ou autres cations, puis traiter la solution résultante sur un échangeur d'ions faiblement basique pour éliminer toute trace d'acide sulfurique, de sulfates ou d'autres impuretés et ainsi obtenir un acide lactique purifié.

Les premiers inconvénients de ce procédé résident dans le coût énergétique de ce système qui met en oeuvre deux électrodialyses en série, et l'importance des volumes manipulés. Le coût énergétique est par exemple directement lié à la nécessité de concentrer les solutions renfermant les sels de l'acide lactique pour effectuer l'électrodialyse fractionnante, ces coûts étant d'autant plus élevés que l'on souhaite récupérer de plus grandes quantités d'acide lactique de haute pureté.

Le second inconvénient est lié au phénomène d'encrassement des membranes des électrodialyseurs.

C'est ainsi que le brevet US 5.681.728 insiste sur la nécessité de mettre en oeuvre une étape de nanofiltration et/ou d'utilisation d'une résine chélatante comme étape préalable obligatoire pour obtenir un fonctionnement correct des électrodialyseurs de concentration ou de fractionnement, sinon le coût de la purification de l'acide lactique est d'autant plus élevé qu'il faut procéder au nettoyage ou au remplacement des membranes d'électrodialyse.

Le brevet US 5.814.498 décrit également la mise en oeuvre d'une étape de nanofiltration du milieu de fermentation clarifié (i.e. débarrassé des microorganismes producteurs d'acide lactique), en amont de l'utilisation de l'électrodialyse fractionnante bipolaire, pour éliminer les cations divalents dudit milieu de fermentation, qui diminuent en effet l'efficacité de l'électrodialyse.

De la même manière, il est décrit dans le brevet CA 2.227.398 de mettre en oeuvre une étape de nanofiltration ou une boucle basique consistant en une colonne de résine cationique pour éliminer les ions multivalents avant de procéder à l'électrodialyse à membranes anionique ou cationique proprement dite.

Les procédés ainsi perfectionnés souffrent cependant tous encore en pratique de la succession d'étapes nombreuses et lourdes, qui rendent la purification de l'acide lactique à partir du milieu de fermentation particulièrement longue et fastidieuse.

De tout ce qui précède, il résulte qu'il existe un besoin non satisfait de disposer d'un procédé moins coûteux, permettant la séparation, la concentration et la purification d'un acide lactique de haute pureté et ce, avec un excellent rendement à partir d'un milieu de fermentation.

Soucieuse de développer un procédé qui permette de répondre mieux que ceux qui existent déjà aux contraintes de la pratique, la société Demanderesse a constaté que cet objectif pouvait être atteint par un procédé combinant sur le milieu de fermentation débarrassé des microorganismes et acidifié dans des conditions particulières, une étape de chromatographie sur résine échangeuse de cations suivie directement d'une étape d'électrodialyse fractionnante.

La société Demanderesse a ainsi vaincu un préjugé technique selon lequel la séparation chromatographique et l'électrodialyse ne peuvent être efficacement et directement combinées pour la préparation d'un acide lactique de haute pureté, car il est admis que chacune de ces méthodes de purification est à considérer indépendamment l'une de l'autre.

C'est ainsi que dans le brevet US 5.641.406, l'étape d'électrodialyse fractionnante supplémentaire qui est envisagée dans le procédé de purification chromatographique de l'acide lactique à partir de son milieu de fermentation, n'est mise en oeuvre que sur les solutions salines diluées pour régénérer la base à partir de ladite solution saline chromatographiée et de la recycler dans le milieu de fermentation pour en réguler le pH. Rien ici n'indique ni ne suggère de réaliser cette électrodialyse sur l'acide lactique libre en équilibre avec ses sels, contenu dans la fraction de chromatographie.

Le procédé de séparation et de purification d'acide lactique à partir d'un milieu de fermentation conforme à l'invention est caractérisé par le fait que l'on :
a) sépare les micro-organismes producteurs d'acide lactique des autres composants du milieu de fermentation,
b) concentre la solution aqueuse renfermant le(s) sel(s) de l'acide lactique ainsi clarifiée,
c) acidifie ladite solution aqueuse concentrée, de préférence avec de l'acide minéral fort, jusqu'à une valeur de pH inférieure ou égale à 3,
d) passe la solution aqueuse ainsi acidifiée sur une résine échangeuse de cations, de manière à obtenir une fraction constituée essentiellement d'acide lactique sous forme libre et d'au plus 25 % en poids de sel(s) de l'acide lactique, exprimé en sec sur le poids sec de ladite solution,
e) concentre éventuellement ladite fraction et la soumet à une électrodialyse fractionnante bipolaire de manière à obtenir une solution aqueuse enrichie en acide lactique,
f) purifie, concentre et récupère l'acide lactique à partir de ladite solution aqueuse enrichie.

Les microorganismes producteurs d'acide lactique sont choisis indifféremment, et notamment dans le groupe constitué des *Lactobacillus, Bacillus, Rhizopus et Streptococcus.* La composition de leur milieu de fermentation est largement décrite dans l'état de la technique.

L'ajustement du pH du milieu de fermentation à une valeur par exemple comprise entre 5,5 et 6,5 s'effectue en alimentant en continu le milieu de fermentation avec une base choisie de préférence dans le groupe constitué par NaOH, Na₂CO₃, NaHCO₃, Ca(OH)₂, CaCO₃, KOH ou NH₄OH.

Ceci conduit à la formation dans le milieu de fermentation de lactate de potassium, de lactate de sodium, de lactate de calcium et de lactate d'ammonium, seuls ou en mélange, suivant la (les) base(s) choisie(s). Il est ainsi possible de réaliser des régulations mixtes avec de la potasse et de l'ammoniac, ou avec du carbonate de magnésium et du carbonate de calcium.

Dans le procédé conforme à l'invention, on préfère avantageusement effectuer la régulation du pH du milieu de fermentation par NaOH ou NH₄OH, ce qui conduit à la formation respectivement de lactate de sodium ou de lactate d'ammonium. Selon l'invention, l'acide lactique contenu dans le milieu de fermentation se trouve partiellement ou totalement, de préférence totalement, sous forme de sel(s).

La première étape du procédé conforme à l'invention consiste à séparer les microorganismes producteurs d'acide lactique des autres composants du milieu de fermentation.

Cette séparation est réalisée par toute technique connue par ailleurs de l'homme du métier et peut consister en une microfiltration, une centrifugation ou une précipitation desdits microorganismes par des agents floculants. Ces techniques peuvent être également combinées.

Dans le procédé conforme à l'invention, il est préféré une technique de microfiltration, en utilisant une membrane de microfiltration dont la porosité est adaptée à la taille des microorganismes considérés, par exemple des membranes TECHSEP de 0,1 µm de porosité pour des microorganismes producteurs d'acide lactique du genre *Lactobacillus.*

La seconde étape du procédé conforme à l'invention consiste à concentrer la solution aqueuses ainsi clarifiée.

Les titres en acide lactique des milieux de fermentation sont classiquement peu élevés, i.e. compris entre 5 et 15 % en poids. Il est donc conseillé de concentrer lesdits milieux de fermentation clarifiés en sel(s) de l'acide lactique avant de poursuivre les étapes de séparation.

Cette étape de concentration peut être réalisée par toute technique connue en soi de l'homme du métier, par exemple en évaporateur continu à une température comprise entre 50 et 65°c, de préférence à une température de l'ordre de 50°c.

Selon le procédé conforme à l'invention, on concentre le milieu de fermentation clarifié en sel(s) de l'acide lactique à une valeur comprise entre 30 et 60 % en poids, de préférence comprise entre 35 et 50 % et par exemple de l'ordre de 40 % en poids.

La troisième étape du procédé conforme à l'invention consiste à acidifier ladite solution aqueuse concentrée, de préférence avec de l'acide minéral fort, jusqu'à une valeur de pH inférieure ou égale à 3.

L'acidification est réalisée de manière à provoquer la dissociation de tout sel de l'acide lactique en acide lactique libre et en sa base correspondante.

Dans le cas où la régulation du pH du milieu de fermentation est effectuée par de l'ammoniac, cette étape d'acidification, réalisée par exemple par de l'acide sulfurique, permettra de dissocier le lactate d'ammonium en acide lactique et sulfate d'ammonium.

En outre, dans le procédé conforme à l'invention, il est choisi d'acidifier le milieu de fermentation clarifié et concentré à une valeur de pH telle que la solution aqueuse résultante contienne encore au plus 25 % de sel(s) de l'acide lactique en équilibre avec l'acide lactique libre ainsi formé, en plus des sulfates libérés.

On acidifie donc le milieu de fermentation clarifié et concentré à une valeur de pH inférieure ou égale à 3, de préférence à une valeur de pH comprise entre 1,5 et 3.

La quatrième étape du procédé conforme à l'invention consiste ensuite à passer la solution aqueuse ainsi acidifiée sur une résine échangeuse de cations, de manière à obtenir une fraction constituée essentiellement d'acide lactique sous forme libre et d'au plus 25 % en poids sec de sel(s) de l'acide lactique, exprimé sur le poids sec de ladite solution.

Le passage sur colonne de séparation chromatographique sur résine échangeuse de cations forts permet de retenir préférentiellement l'acide lactique et le(s) sel(s) de l'acide lactique contenus dans ladite solution acidifié.

Cette étape peut avantageusement être menée sur une résine échangeuse de cations fortement acide du type polystyrène acide sulfonique, réticulé par au moins 4 %, de préférence au moins 7 %, de divinylbenzène.

L'élution à l'eau permet d'éliminer dans les premières fractions la quasi totalité des impuretés du milieu de fermentation. Celles-ci sont constituées principalement par les sucres et protéines résiduels non consommés, et par les sels inorganiques de type ions multivalents (calcium, magnésium) et toute base correspondant à tout sel de l'acide lactique dissocié. Dans le cas où la solution de lactate d'ammonium est traitée à l'acide sulfurique, il s'agira de sulfate d'ammonium.

Les fractions suivantes renferment l'acide lactique sous forme libre et au plus 25 % en poids sec de sel(s) de l'acide lactique, exprimé sur le poids sec de ladite solution.

La société Demanderesse a ainsi noté que le choix de ce rapport acide lactique libre sur sel(s) de l'acide lactique, obtenu de par l'acidification de la solution à chromatographier dans les conditions de l'étape précédemment décrite, est choisi de manière à ce que les pertes en acide lactique libre dans les premières fractions d'élution, i.e. avec les impuretés, soient limitées au maximum, comme il sera exemplifié ci-après.

De plus, et c'est ce qui constitue un autre avantage du procédé conforme à l'invention, la consommation en acide sulfurique sera d'autant plus faible qu'il n'est pas nécessaire de trop acidifier le milieu de fermentation clarifié.

La cinquième étape du procédé conforme à l'invention consiste, après concentration éventuelle, à soumettre ladite fraction à une électrodialyse fractionnante bipolaire de manière à obtenir une solution aqueuse enrichie en acide lactique.

La fraction renfermant l'acide lactique et au plus 25 % de sel(s) de l'acide lactique est donc soumise à une électrodialyse fractionnante bipolaire de manière à dissocier tout sel de l'acide lactique résiduel en acide lactique libre et en sa base correspondante.

Ladite fraction peut être concentrée à une matière sèche de l'ordre de 15 à 25 %, par tout moyen connu de l'homme du métier, par exemple par évaporation à une température comprise entre 50 et 60 °C.

En pratique l'électrodialyse est effectuée dans un dispositif constitué d'au moins deux compartiments et d'au moins deux membranes bipolaires, ledit dispositif pouvant en outre contenir au moins une autre membrane, cationique ou anionique.

Le dispositif d'électrodialyse bipolaire est par exemple constitué de sept cellules constituées chacune par deux membranes bipolaires et d'une membrane cationique.

Les conditions opératoires sont conduites de manière à ce que moins de 50 meq de cations, par exemple ammonium, ne subsistent dans la solution électrodialysée.

La dernière étape du procédé conforme à l'invention consiste à purifier, concentrer et récupérer l'acide lactique à partir de ladite solution aqueuse.

Cette dernière étape est réalisée de manière à éliminer les dernières impuretés présentes dans la fraction ainsi électrodialysée enrichie en acide lactique libre, impuretés composées principalement de sucres résiduels (de l'ordre de 0,2 à 0,3 %), les ions, par exemple ammonium, que l'électrodialyse n'a pas permis d'éliminer complètement et les sulfates résiduels.

Il est ainsi choisi avantageusement de :
a) traiter ladite solution aqueuse électrodialysée à une température inférieure à 180 °C, de préférence comprise entre 100 et 150 °C, pendant 15 min à 1 h 30,
b) passer la solution ainsi traitée thermiquement sur une colonne de noir,
c) déminéraliser la solution ainsi obtenue sur une résine cationique forte, puis une résine anionique moyennement basique,
d) concentrer l'acide lactique ainsi purifié.
La première étape permet de carboniser les sucres résiduels. On peut choisir, comme il sera exemplifié ci-après, de chauffer à une température de 120°C pendant 1 h.

La seconde étape consiste à décolorer la solution ainsi chauffée, sur colonne de noir.

La troisième étape permet d'éliminer l'ensemble des sels inorganiques par passage sur deux résines cationique forte puis moyennement basique.

Enfin, il est procédé à la concentration de la solution résultante de manière à l'amener à une matière sèche au moins égale à 45 % en poids, généralement compris entre 50 et 90 %.

De manière surprenante et inattendue, l'analyse de l'acide lactique ainsi séparé et purifié a révélé une qualité conforme aux normes pharmaceutiques FCC, comme il sera exemplifié ci-après.

D'autres caractéristiques et avantages de l'invention apparaîtront à la lecture des exemples qui suivent. Ils ne sont toutefois donnés ici qu'à titre illustratif et non limitatif.

### EXEMPLE

Un milieu de fermentation renfermant 78 g/l (exprimé en acide lactique) de lactate d'ammonium, produit par un microorganisme du genre *Lactobacillus*, est soumis à une microfiltation sur membrane TECHSEP de porosité 0,1 µm de manière à en éliminer les cellules.

Ce milieu ainsi clarifié est concentré sur un évaporateur sous vide à 50°C de manière à ce que sa concentration en lactate d'ammonium soit de 40 %.

La solution aqueuse concentrée obtenue est acidifiée à un pH de 2,0 environ par ajoût d'acide sulfurique pur, de manière à présenter un rapport pondéral acide lactique / lactate d'ammonium de 85/15.

Cette solution concentrée et acidifiée est ensuite soumise à une séparation chromatographique sur résine échangeuse de cations forts constituée de polystyrène acide sulfonique réticulé par 7 % de divinylbenzène.

Elle est alimentée à un débit de 50 l/h.

L'élution à l'eau (280 l/h) permet d'éliminer les impuretés constituées de protéines et sucres non consommés, de sulfate d'ammonium et de cations divalents tels que Ca⁺⁺ et Mg⁺⁺, en une première fraction.

La seconde fraction contenant l'acide lactique sous forme libre en équilibre, appauvrie en sulphate d'ammonium, est éluée à un débit de 130 l/h pour une concentration de 10 % en poids dans la fraction finale.

Le rendement de chromatographie est de 97 %.

Ladite fraction, concentrée à 20 % en poids, est soumise ensuite une électrodialyse frationnante.

L'électrodialyseur consiste en un dispositif à membranes TOKOYAMA constitué de 7 cellules, commercialisé par la Société EURODIA et équipé de membranes bipolaires et cationiques.

On utilise une configuration combinant 200 cm² de surface membranaire bipolaire et une intensité de courant de 100 mA/cm² soit, en tout, de 20 A.

3 litres de la fraction susmentionnée concentrée à 20 % et contenant 600g de mélange acide lactique / lactate d'ammonium et des impuretés résiduelles (sucres, protéines, impuretés minérales), sont passés dans ledit dispositif et ce, pendant 10 minutes jusqu'à atteindre une tension finale de 23V.

La fraction de départ qui contenait 2 équivalents(eq) d'acide lactique pour 300 milliéquivalents (meq) d'ions NH₄⁺ et 300 meq d'ions SO₄²⁻, renferme, après électrodialyse, 2 éq d'acide lactique, 300 meq d'ions SO₄²⁻ mais seulement 50 meq d'ions NH₄⁺.

La fraction électrodialysée résultante, enrichie donc en acide lactique, est ensuite purifiée par un traitement thermique à 120°C pendant 1h afin d'éliminer les traces de protéines et sucres résiduels (0,2 % en poids) qui n'ont pas été éliminés lors de l'étape antérieure de séparation chromatographique.

Cette carbonisation conduit à la formation de matières colorantes qui sont ensuite éliminées sur colonne de noir.

On procède finalement à une étape de déminéralisation pour éliminer les ions NH₄⁺, Na⁺ et SO₄²⁻ encore présents et ce, par passage sur résine cationique forte puis sur résine moyennement basique de type acrylique.

La solution ainsi obtenue renferme 99,5 %, exprimé en sec/sec, d'acide lactique de haute pureté. Elle est concentrée à une matière sèche (MS) de 90 % par évaporation à 50°C.

Le produit obtenu présente l'avantage d'être parfaitement conforme aux normes FCC, en particulier en termes de pureté et de stabilité.

## Revendications

1. Procédé de séparation et de purification d'acide lactique à partir d'un milieu de fermentation contenant ledit acide totalement ou partiellement sous forme de sel(s), **caractérisé par le fait que** l'on :
a) sépare les micro-organismes producteurs d'acide lactique des autres composants du milieu de fermentation,
b) concentre la solution aqueuse renfermant le(s) sel(s) de l'acide lactique ainsi clarifiée,
c) acidifie ladite solution aqueuse concentrée, de préférence avec de l'acide minéral fort, jusqu'à une valeur de pH inférieure ou égale à 3,
d) passe la solution aqueuse ainsi acidifiée sur une résine échangeuse de cations, de manière à obtenir une fraction constituée essentiellement d'acide lactique sous forme libre et d'au plus 25 % en poids de sel(s) de l'acide lactique, exprimé en sec sur le poids sec de ladite solution,
e) concentre éventuellement ladite fraction et la soumet à une électrodialyse fractionnante bipolaire de manière à obtenir une solution aqueuse enrichie en acide lactique,
f) purifie, concentre et récupère l'acide lactique à partir de ladite solution aqueuse enrichie.

2. Procédé selon la revendication 1, **caractérisé par le fait que** ledit milieu de fermentation renferme au moins un sel de l'acide lactique choisi dans le groupe constitué par le lactate de potassium, le lactate de sodium, le lactate de calcium, le lactate d'ammonium et leurs mélanges, de préférence au moins du lactate d'ammonium ou du lactate de sodium.

3. Procédé selon l'une ou l'autre des revendications 1 et 2, **caractérisé par le fait que** l'étape a) de séparation est choisie dans le groupe constitué par la microfiltration, la centrifugation et la précipitation par des agents floculants et, de préférence, consiste en la microfiltration.

4. Procédé selon l'une quelconque des revendications 1 à 3, **caractérisé par le fait que** l'on acidifie la solution aqueuse concentrée obtenue à l'étape b) à une valeur de pH comprise entre 1,5 et 3.

5. Procédé selon l'une quelconque des revendications 1 à 4, **caractérisé par le fait que** l'acide minéral fort utilisé à l'étape c) est l'acide sulfurique.

6. Procédé selon l'une quelconque des revendications 1 à 5, **caractérisé par le fait que** la résine échangeuse de cations utilisée lors de l'étape d) est une résine échangeuse de cations fortement acide du type polystyrène acide sulfonique, réticulé par au moins 4 %, de préférence par au moins 7 %, de divinylbenzène.

7. Procédé selon l'une quelconque des revendications 1 à 6, **caractérisé par le fait que** l'électrodialyse est effectuée dans un dispositif constitué d'au moins deux compartiments et d'au moins deux membranes bipolaires, ledit dispositif pouvant contenir en outre au moins une membrane cationique ou anionique.

8. Procédé selon l'une quelconque des revendications 1 à 7, **caractérisé par le fait que**, lors de l'étape f), la purification et la concentration de l'acide lactique à partir de la solution aqueuse électrodialysée enrichie en acide lactique, consiste à :
a) traiter ladite solution aqueuse électrodialysée à une température inférieure à 180 °C, de préférence comprise entre 100 et 150 °C, pendant 15 min à 1 h 30,
b) passer la solution ainsi traitée thermiquement sur une colonne de noir,
c) déminéraliser la solution ainsi obtenue sur une résine cationique forte, puis une résine anionique moyennement basique,
d) concentrer l'acide lactique ainsi purifié.

## Claims

1. Process for the separation and purification of lactic acid from a fermentation medium containing said acid totally or partially in the form of salt(s), **characterised in that** :
a) the lactic acid-producing micro-organisms are separated from the other components of the fermentation medium;
b) the resulting clarified aqueous solution containing the lactic acid salt(s) is concentrated;
c) said concentrated aqueous solution is acidified, preferably with a strong mineral acid, to a pH below or equal to 3;
d) the resulting acidified aqueous solution is passed over a cation exchange resin to give a fraction consisting essentially of lactic acid in the free form and at most 25% by weight of lactic acid salt(s), expressed by dry weight relative to the dry weight of said solution;
e) said fraction is optionally concentrated and is subjected to bipolar fractionating electrodialysis to give an aqueous solution enriched in lactic acid; and
f) the lactic acid is purified, concentrated and recovered from said enriched aqueous solution.

2. Process according to claim 1, **characterised in that** said fermentation medium contains at least one lactic acid salt selected from the group consisting of potassium lactate, sodium lactate, calcium lactate, and ammonium lactate and their mixtures, preferably ammonium lactate or sodium lactate.

3. Process according to anyone of claims 1 and 2, **characterised in that** the separation step a) is selected from the group consisting of microfiltration, centrifugation and precipitation with flocculants, preferably microfiltration.

4. Process according to anyone of claims 1 to 3, **characterised in that** the concentrated aqueous solution obtained in step b) is acidified to a pH of between 1.5 and 3.

5. Process according to anyone of claim 1 to 4, **characterised in that** the strong mineral acid used in step c) is sulfuric acid.

6. Process according to anyone of claims 1 to 5, **characterised in that** the cation exchange resin used in step d) is a strongly acidic cation exchange resin of the polystyrenesulfonic acid type crosslinked with at least 4%, preferably at least 7%, of divinylbenzene.

7. Process according to anyone of claims 1 to 6, **characterised in that** the electrodialysis is carried out in a device consisting of at least two compartments and at least two bipolar membranes, and said device may further contain at least one cationic or anionic membrane.

8. Process according to anyone of claims 1 to 7, **characterised in that**, in step f), the purification and concentration of the lactic acid from the electrodialyzed aqueous solution enriched in lactic acid consists in:
a) heat treating said electrodialyzed aqueous solution at a temperature below 180°C, preferably between 100 and 150°C, for 15 min to 1h30;
b) passing the resulting heat-treated solution through a column of charcoal;
c) demineralizing the resulting solution on a strong cationic resin and then a moderately basic anionic resin; and
d) concentrating the resulting purified lactic acid.

## Patentansprüche

1. Verfahren zur Trennung und Reinigung von Milchsäure aus einem Fermentationsmedium, das diese Säure vollständig oder teilweise in Form von Salz(en) enthält, **dadurch gekennzeichnet, daß** man
a) die Milchsäure erzeugenden Mikroorganismen von den anderen Bestandteilen des Fermentationsmediums trennt,
b) die wässrige Lösung, die das/die Salz/e der auf diese Weise geklärten Milchsäure enthält, konzentriert,
c) die konzentrierte wässrige Lösung vorzugsweise mit starker Mineralsäure bis zu einem pH-Wert von weniger als oder gleich 3 ansäuert,
d) die auf diese Weise angesäuerte wässrige Lösung so über ein Kationenaustauschharz leitet, daß man eine Fraktion erhält, die im wesentlichen aus Milchsäure in freier Form und aus höchstens 25 Gew.-% Salz(en) der Milchsäure besteht, ausgedrückt trocken bezogen auf das Trockengewicht der Lösung,
e) ggf. diese Fraktion konzentriert und sie einer bipolaren fraktionierenden Elektrodialyse so unterzieht, daß man eine mit Milchsäure angereicherte wässrige Lösung erhält,
f) die Milchsäure aus der angereicherten wässrigen Lösung reinigt, konzentriert und gewinnt.

2. Verfahren nach Anspruch 1, **dadurch gekennzeichnet, daß** das Fermentationsmedium mindestens ein Salz der Milchsäure enthält, das aus der Gruppe ausgewählt ist, die aus Kaliumlactat, Natriumlactat, Calciumlactat, Ammoniumlactat und ihren Mischungen besteht, und zwar vorzugsweise mindestens Ammoniumlactat oder Natriumlactat.

3. Verfahren nach einem der Ansprüche 1 und 2, **dadurch gekennzeichnet, daß** der Schritt a) der Trennung aus der Gruppe ausgewählt wird, die aus Mikrofiltration, Zentrifugierung und Ausfällung mit Hilfe von Ausflockungsmitteln besteht, und vorzugsweise in der Mikrofiltration besteht.

4. Verfahren nach einem der Ansprüche 1 bis 3, **dadurch gekennzeichnet, daß** man die in Schritt b) erhaltene konzentrierte wässrige Lösung auf einen pH-Wert zwischen 1,5 und 3 ansäuert.

5. Verfahren nach einem der Ansprüche 1 bis 4, **dadurch gekennzeichnet, daß** die in Schritt c) verwendete starke Mineralsäure Schwefelsäure ist.

6. Verfahren nach einem der Ansprüche 1 bis 5, **dadurch gekennzeichnet, daß** das in dem Schritt d) verwendete Kationenaustauschharz ein stark saures Kationenaustauschharz vom Typ Polystyrolsulfonsäure ist, die mit mindestens 4%, vorzugsweise mindestens 7% Divinylbenzol vernetzt ist.

7. Verfahren nach einem der Ansprüche 1 bis 6, **dadurch gekennzeichnet, daß** die Elektrodialyse in einer Vorrichtung durchgeführt wird, die aus mindestens zwei Kammern und aus mindestens zwei bipolaren Membranen besteht, wobei diese Vorrichtung außerdem mindestens eine kationische oder anionische Membran enthalten kann.

8. Verfahren nach einem der Ansprüche 1 bis 7, **dadurch gekennzeichnet, daß** im Schritt f) die Reinigung und die Konzentration der Milchsäure aus der mit Milchsäure angereicherten, elektrodialysierten wässrigen Lösung darin bestehen, daß man
a) die elektrodialysierte wässrige Lösung während 15 min bis 1 h 30 mit einer Temperatur unter 180°C, vorzugsweise zwischen 100 und 150°C, behandelt,
b) die auf diese Weise thermisch behandelte Lösung über eine Ruß-Säule führt,
c) die auf diese Weise erhaltene Lösung auf einem starken kationischen Harz und dann einem mittel basischen anionischen Harz entmineralisiert,
d) die auf diese Weise gereinigte Milchsäure konzentriert.
